# EUROPÄISCHE PATENTANMELDUNG

(11) **EP 0 647 847 A1**
(43) Veröffentlichungstag der Anmeldung: **12.04.1995**
(21) Anmeldenummer: 94112700.3
(22) Anmeldetag: 13.08.1994
(51) Int. Cl.: G01N 27/64, G01N 33/44

(54) **Verwendung eines Massenspektrometers mit Sekundärionisation zur Inspektion von Behältern**

(30) Priorität: 14.09.1993 CH 2747/93
(71) Anmelder: ELPATRONIC AG, CH-6303 Zug (CH)
(72) Erfinder: Federer, Werner, Dr., A-6060 Tulfes (AT); Robertson, Peter Murday, CH-8185 Winkel (CH); Villinger, Johannes, Dr., A-6020 Innsbruck (AT)

(57) **Zusammenfassung**

Zur Prüfung von PET-Flaschen auf Verunreinigungen wird ein Massenspektrometer verwendet, bei dem die Gasprobe von einem ionisierten Primärgas ionisiert wird. Dies bewirkt eine wesentlich geringere oder eine eindeutige Fragmentation der Gasprobe. Dadurch ist das entstehende Spektrum ohne Zusatzinformation sehr aussagekräftig und kann sehr rasch ausgewertet werden, was erst den Einsatz eines Massenspektrometers in der industriellen Inspektion ermöglicht.

## Beschreibung

Die Erfindung betrifft die Verwendung eines Massenspektrometers mit einer Anordnung zur selektiven Vorbehandlung des zu untersuchenden Gasgemisches zur Inspektion von auf einer Förderanlage geförderten Behältern, wobei jedem Behälter mindestens eine Gasprobe entnommen und deren Massenspektrum bestimmt und ausgewertet wird.

Ein Massenspektrometer, bei welchem das zu untersuchende Gasgemisch durch Primärionen von bestimmter innerer Energie ionisiert wird, ist aus dem Dokument EP-B-290 711 bekannt. Erfindungsgemäss wird nun vorgeschlagen, ein derartiges Massenspektrometer bei der Inspektion von Behältern einzusetzen. Ein solches Massenspektrometer hat den Vorteil, dass aufgrund der sehr genau einstellbaren "sanften" Ionisation der dem Behälter entnommenen Gasprobe entweder keine Fragmentation derselben stattfindet oder jedenfalls eine hochgenau definierte. Dies hat den Vorteil, dass das entstehende Massenspektrum gut und in sinnvoller Zeit auswertbar ist. Dadurch wird der Einsatz eines Massenspektrometers auch in industriell betriebenen Inspektionsanlagen mit hohem Behälterdurchsatz möglich, mit den sich daraus ergebenden Vorteilen der genauen Bestimmung von Stoffen oder Stoffresten in den Behältern. Die genaue und rasche Bestimmung des Spektrums bei geringer Fragmentation mit einem solchen Massenspektrometer erlaubt insbesondere die rasche Auswertung des erzeugten Spektrums durch Vergleich mit gespeicherten Spektren in einer "Spektren-Bibliothek". Der Vergleich kann durch einen Muster-Vergleich mittels eines Rechners erfolgen. Zum Vergleich der gespeicherten Spektren mit dem jeweils erzeugten Spektrum können auch sogenannte "neuronale Netze" oder mit sogenannter "unscharfer Logik" (fuzzy logic) arbeitende Vergleichseinrichtungen verwendet werden.

Vorzugsweise erfolgt die Verwendung des beschriebenen Massenspektrometers bei der Inspektion von Mehrweg-PET-Flaschen.

Die Inspektion auf Fremdstoffe vor dem Waschprozess ist für die Mehrweg-PET-Flasche unerlässlich, da sich diese Flasche auch sehr gut für die Aufbewahrung von Fremdstoffen aller Art eignet. So können z.B. Salatsaucen, Reinigungsmittel, Benzin, Motorenöl, verschiedenste Lösungsmittel wie Methanol, Aceton und hochprozentige Spirituosen in diesem verbraucherfreundlichen Gebinde gelagert worden sein. Alle diese Fremdstoffe können Probleme verursachen. Selbst nach mehrmaligem Waschen und Spülen verändern bestimmte Fremdstoffe den Geschmack des frisch abgefüllten Produktes oder machen das Getränk sogar ungeniessbar. Zwar sind die verbleibenden Fremdstoffkonzentrationen nach dem Waschprozess so klein, dass eine gesundheitliche Schädigung des Konsumenten praktisch ausgeschlossen ist. Trotzdem ist die sichere Erkennung aller Fremdstoffe wesentlich. Undefinierbare Fremdgerüche in frischen Produkten führen unweigerlich zu Konsumentenbeschwerden.

Die Erfindung, welche grundsätzlich für Behälter jeglicher Art anwendbar ist, wird daher nachfolgend anhand einer PET-Flaschen Inspektionsmaschine beispielhaft näher erläutert. Dabei zeigt
Figur 1 in stark schematisierter Form eine Inspektionsmaschine mit dem speziellen Massenspektrometer; und
Figur 2 ein Spektrum.

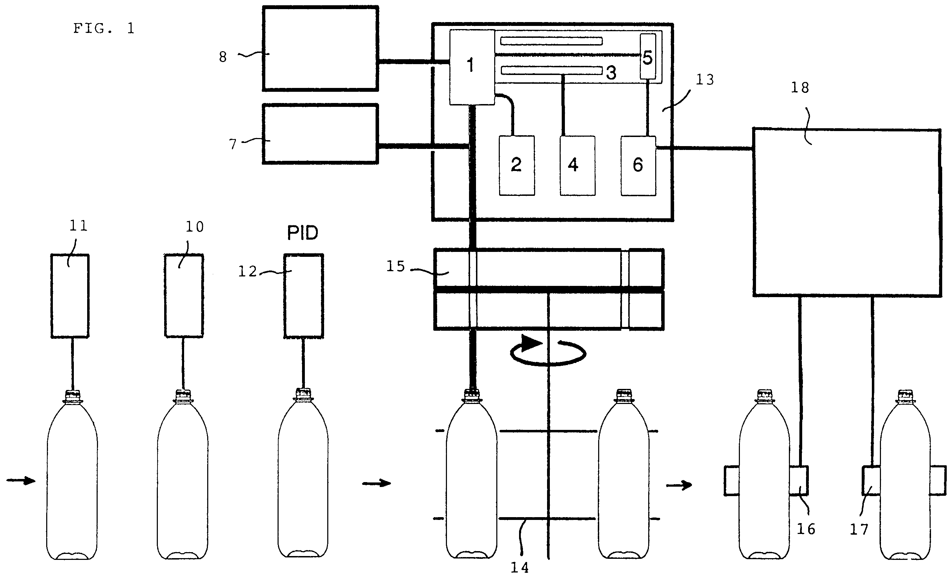

Das Prinzip der Massenspektroskopie ist grundsätzlich sehr einfach und schematisch in Figur 1 ersichtlich: Der Flasche wird ein Probegas entnommen und über mehrere Stufen auf einen relativ kleinen Druck von ca. 10⁻⁵ bis 10⁻⁶ bar abgepumpt. Dafür sind zwei ölfreie, wartungsarme Membranpumpen 7 und eine ebenfalls ölfreie Turbomolekularpumpe 8 vorgesehen. Dem nachfolgenden Ionisierungsprozess des Probegases muss sehr viel Bedeutung beigemessen werden, da die Moleküle des Probegases und auch der Luft (Sauerstoff, Stickstoff, Wasser, Kohlendioxyd) sonst fragmentiert werden. Damit würde sehr viel Information über die gemessene Substanz verloren gehen. Es ist wichtig, dass bei der raschen industriellen Inspektion das aufgenommene Spektrum eindeutig ist. Aus dem in der Gasprobe enthaltenen H₂0 sollten z.B. nur positive H₂0-Ionen gebildet werden und nicht OH-Ionen mit der Massezahl 17, da auch Ammoniak (NH₃) die Massezahl 17 aufweist und für die rasche, industrielle Auswertung des Spektrums es wesentlich ist, dass der dort ersichtliche Peak nur vom Ammoniak stammt. Die sich bei der industriellen Anwendung der Massenspektroskopie stellenden Probleme sind somit deutlich verschieden von Laboranwendungen, bei denen genügend Zeit für zusätzliche, differenzierende Auswertungen besteht und somit bei dem genannten Beispiel das Auftreten von OH-Ionen ohne weiteres zulässt.

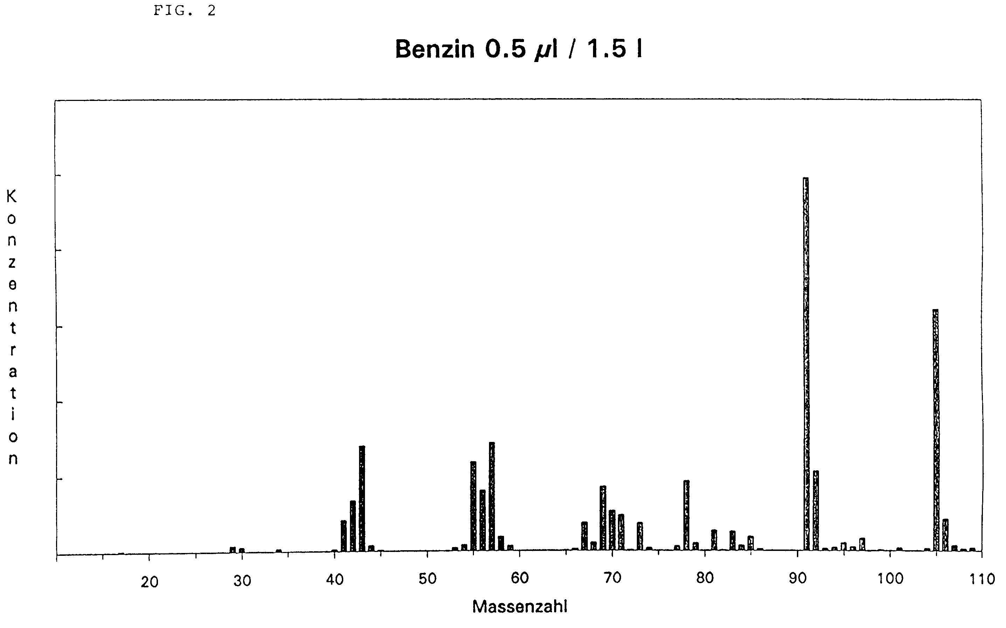

Das Spektrum in Figur 2 zeigt die Menge von Informationen, die über die Messung einer einzelnen Flasche in weniger als 100 Millisekunden erhalten wurden (Inspektionrate z.B. 600 Flaschen pro Minute). Die schnelle Auswertung der Spektren erfolgt vorzugsweise durch einen Vergleich. Der schnelle Erkennungsalgorithmus basiert auf einer Bibliothek mit den wichtigsten Substanzklassen. Die Messdaten werden aufbereitet und mit der Bibliothek verglichen. Wenn die gemessenen Daten genügend nahe bei schon bekannten Substanzklassen liegen, wird die Flasche entsprechend den Vorgaben ausgeworfen oder auf der Linie behalten. Der Algorithmus ist vorzugsweise so ausgelegt, dass auch unbekannte Substanzen oder Gemische von bekannten Substanzen richtig beurteilt werden. Es reicht zum Beispiel, Benzin als Substanzklasse in der Bibliothek gespeichert zu haben, um Motorenöl und Diesel richtig zu beurteilen und auszuwerfen. Unbekannte Substanzen können jedoch auch zusätzlich sehr einfach mittels einer Lernfunktion registriert und gespeichert werden. Ein Orangengetränk lässt sich auch ohne weiteres von einem Zitronengetränk unterscheiden. Bei Verwendung von Multiproduktflaschen kann also eine Geschmackübertragung durch vorhergehende Produkte vermieden werden.

Die Entscheidung, ob eine Flasche ausgeworfen wird, basiert nicht wie bei herkömmlichen Systemen auf einem Informationsmangel, sondern auf einer positiven Erkennung einer Substanz, sei es eine harmlose, die durchgelassen, oder ein störende, die ausgeworfen wird.

Jedes empfindliche Messinstrument hat über eine gewisse Zeit ein bestimmtes Erinnerungsvermögen, d.h. eine bestimmte endliche Abfallzeit des Messignals nach einem starken Ausschlag. Dieser sogenannte Memory-Effekt hat üblicherweise Falschauswürfe zur Folge, da das System nicht zwischen einer vermeintlichen Kontamination der vorhergehenden Flasche und einer wirklichen Kontamination der momentan gemessenen Flasche unterscheiden kann. Das Massenspektrometer liefert jedoch so differenzierte Informationen, dass auch dieses Problem ohne weiteres lösbar ist. Falls nachfolgende Flasche die gleiche Art Kontamination mit entsprechend geringerer Konzentration aufweisen, ist die Wahrscheinlichkeit sehr gross, dass es sich um Memory-Flaschen handelt. Diese Flaschen werden darum in einen Repass-Container ausgeworfen oder speziell ausgeleitet. Dadurch wird eine nochmalige Prüfung der Flaschen ermöglicht.

Die Maschinen für die Fremdstoffinspektion sind vorzugsweise als Drei-Karussell-Maschinen mit Einlauf- und Auslaufschnecke konzipiert. In der Einlaufschnecke werden die Flaschen auf grössere Mengen Restflüssigkeit und auf nicht entdeckelte Flaschen überprüft (Prüfstationen 10, 11). Anschliessend werden die Flaschen im Durchlauf mittels eines Fotoionisationsverfahrens (PID) von einem Hochgeschwindigkeits-PID-Messkopf 12 geprüft. Der PID-Messkopf ist auf einen relativ hohen Schwellenwert eingestellt, so dass nur hochkontaminierte Flaschen, z.B. gesättigte Benzinkonzentrationen, erfasst werden und keine Falschauswürfe möglich sind. Diese erste Vorkontrolle stellt sicher, dass keine stark verunreinigten Flaschen vom hochempfindlichen Massenspektrometer 13 geprüft werden. So kann der Memory-Effekt von vornherein weitgehend ausgeschlossen werden. Höhere Konzentrationen, die vom PID-Sensor erkannt werden, aber trotzdem einen Memory-Effekt verursachen, werden wie beschrieben von der Spektren-Erkennungssoftware erkannt. Alle Flaschen, die bereits in der Einlaufschnecke erfasst werden (starke Verunreinigung, Verschlüsse, Restflüssigkeit), werden vom Massenspektrometer 13 nicht überprüft und beim Maschinenauslauf in verschiedene Container ausgeworfen oder ausgeleitet.

Für das Massenspektrometer ist das Hauptkarussell 14 mit 16 Stationen vorgesehen. Messröhrchen werden berührungslos in die Flaschen abgesenkt und entnehmen Luftproben, welche eine kleine Konzentration von Getränkerückständen und möglicherweise Verunreinigungen enthalten. Die Gasproben werden über einen Verteilkopf 15 in das Massenspektrometer 13 eingesogen und sequentiell analysiert. Falls der Durchsatz 18.000 Flaschen übersteigt, werden die Flaschen alternierend von zwei verschiedenen Massenspektrometern analysiert. Die Empfindlichkeitsgrenze für Benzin liegt um 0.01 Mikroliter pro 1.5 Liter. Ammoniak kann bis zu einer Grenze von ca. 5 Gewichts-ppm detektiert werden.

Die Flaschen werden danach durch das Auslaufkarussell in die Auslaufschnecke geführt. Dort befinden sich zwei verschiedene Auswurfstationen 16 und 17, welche noch verwendbare und nicht mehr verwendbare Flaschen trennen. Nebst den mit Flüssigkeit gefüllten sowie den noch verschlossenen Flaschen werden auch alle Memory-Flaschen zur nochmaligen Prüfung in den Behälter für wiederverwendbare Flaschen ausgeworfen. Anstelle von Auswurfstationen können optional auch Ausleit- und Rückleitsysteme verwendet werden.

Die Auswurfstationen werden von der Steuereinrichtung 18 angesteuert, die aufgrund des vom Massenspektrometers 13 ermittelteten Spektrums die verunreinigten Flaschen erkennt und ausscheidet. Das Massenspektrometer 13 ist auf bekannte Weise mit einer Ionisationskammer 1 versehen, in welcher die Gasprobe mittels des Ionisationsgases aus dem Behälter 2 ionisiert wird. Durch eine an eine HF-Versorgung 4 angeschlossenen Ablenkeinheit 3 werden die Ionen an den Detektor 5 geführt und von Zähler 6 ausgewertet. Wie bereits erwähnt, gewährleistet die sanfte Ionisierung der Moleküle die differenzierte und empfindliche Messung der gewünschten Substanzen. Für die Ionisierung der Probegasmolekühle wird das Edelgas 2 konventionell mit Elektronen ionisiert. Die Edelgasionen werden über Masse- und Geschwindigkeitsfilter selektiert, so dass nur noch Ionen mit einer ganz bestimmten Energie, nämlich der diskreten Ionisationsenergie, mit dem Probegas reagieren können. Mit diesem Ionisationsprozess ist garantiert, dass Wasser-, Sauerstoff- und Stickstoffmoleküle nicht aufgebrochen werden und daher der ganze untere Messbereich freei bleibt. Damit ist auch die Messung von Ammoniak (Masse 17) gewährleistet. Die Probegasmoleküle durchlaufen nun ein elektromagnetisches Feld, das die Ionen proportional zu ihrem Gewicht oder ihrer Masse ablenkt. Das elektromagnetische Feld wird so gesteuert, dass zur gleichen Zeit immer nur eine bestimmte Masse auf den Detektor 5 treffen kann. Dort werden proportional zu den auftreffenden Ionen elektrische Impulse generiert, die über eine Signalverarbeitung von der Spektren-Erkennungssoftware ausgewertet werden. Das Messergebnis ist somit nicht nur ein einzelner Wert für eine nicht spezifizierbare Verunreinigung. Aufgrund des Spektrums kann nämlich sowohl die Konzentration als auch grundsätzlich die Substanz selbst ermittelt werden. Durch die sanfte Ionisierung ist das Spektrum ohne Zusatzinformation sehr aussagekräftig und kann sehr rasch ausgewertet werden, was überhaupt erst den einsatz eines Massenspektrometers in der industriellen Inspektion ermöglicht.

## Patentansprüche

1. Verwendung mindestens eines Massenspektrometers mit einer Anordnung zur selektiven Vorbehandlung des zu untersuchenden Gasgemisches zur Inspektion von auf einer Förderanlage geförderten Behältern, wobei jedem Behälter mindestens eine Gasprobe entnommen und deren Massenspektrum bestimmt und ausgewertet wird.

2. Verwendung nach Anspruch 1, dadurch gekennzeichnet, dass bei der Auswertung das bestimmte Massenspektrum durch eine Vergleichseinrichtung mit einer Vielzahl gespeicherter Massenspektren verglichen wird.

3. Verwendung nach Anspruch 1 oder 2, dadurch gekennzeichnet, dass aufgrund der Auswertung der jeweilige Behälter einer von mindestens drei Gruppen zugeordnet wird, nämlich einer ersten Gruppe von als gut befundenen Behältern oder einer zweiten Gruppe von auszuscheidenden Behältern oder einer dritten Gruppe von erneut zu prüfenden Behältern.

4. Verwendung nach einem der Ansprüche 1 bis 3, dadurch gekennzeichnet, dass die Behälter Getränke- oder Lebensmittelbehälter oder Behälter für pharmazeutische Produkte sind.

5. Verwendung nach einem der Ansprüche 1 bis 4, dadurch gekennzeichnet, dass die Behälter Mehrweg-PET-Flaschen sind.

6. Verwendung nach einem der Ansprüche 1 bis 5, dadurch gekennzeichnet, dass die jeweils zu untersuchende Gasprobe zur selektiven Vorbehandlung unter Einzelstossbedingungen im zumindest weitgehenden Vakuum mittels Primärionen ionisiert wird, wobei die verwendeten Primärionen Edelgasionen, vorzugsweise Xenon-Ionen sind und die massenspektrometrische Untersuchung an der so vorbereiteten Gasprobe vorgenommen wird.

7. Verwendung nach Anspruch 6, dadurch gekennzeichnet, dass die Ionisationsenergie ca. 12 eV beträgt.

8. Verwendung nach einem der Ansprüche 1 bis 7, dadurch gekennzeichnet, dass die Vorbehandlung der Gasprobe derart erfolgt, dass aus in der Gasprobe enthaltenen H₂O-Molekülen im wesentlichen nur positive H₂0-Ionen gebildet werden und keine OH-Ionen mit der Massezahl 17.

9. Inspektionsvorrichtung für Behälter, insbesondere Mehrweg-PET-Flaschen, welche in einer Fördereinrichtung durch die Vorrichtung bewegt werden, gekennzeichnet durch mindestens ein Massenspektrometer (13) mit einer Anordnung zur Vorbehandlung des zu untersuchenden Gasgemisches, in welchem jeweils eine Gasprobe aus den Behältern prüfbar und deren Massenspektrum ermittelbar ist, und eine Vergleichseinrichtung (18), in welcher das ermittelte Massenspektrum mit einer Vielzahl von gespeicherten Massenspektren vergleichbar ist, um zu bestimmen, ob der jeweilige Behälter mit Fremdstoffen kontaminiert ist.

10. Inspektionsvorrichtung nach Anspruch 9, dadurch gekennzeichnet, dass durch die Vorbehandlung eine hochdefinierte, reproduzierbare Fragmentierung oder keine Fragmentierung der Gasprobenmoleküle erfolgt, derart, dass aus H₂0-Molekülen der Gasprobe im wesentlichen nur positive H₂0-Ionen gebildet werden und keine OH-Ionen.
